# EUROPEAN PATENT SPECIFICATION

(11) **EP 4 065 184 B1**
(45) Date of publication and mention of the grant of the patent: **30.10.2024**
(21) Application number: 20824893.0
(22) Date of filing: 25.11.2020
(51) Int. Cl.: A61L 27/24, A61L 27/46, A61L 27/48, A61L 27/54, A61L 27/56

(54) **A COLLAGEN SCAFFOLD**
KOLLAGENGERÜST
ÉCHAFAUDAGE DE COLLAGÈNE

(30) Priority: 27.11.2019 GB 201917246
(43) Date of publication of application: 05.10.2022
(73) Proprietor: Royal College of Surgeons in Ireland, Dublin 2 (IE)
(72) Inventor: O'BRIEN, Fergal, Dublin 2 (IE); WIDAA, Amro, Dublin 2 (IE); LOPEZ-NORIEGA, Adolfo, Dublin 2 (IE); RYAN, Alan, Dublin 2 (IE); SHEEHY, Eamon, Dublin 2 (IE)
(74) Representative: Purdylucey Intellectual Property
(86) International application number: PCT/EP2020/083428
(87) International publication number: WO 2021/105248

(56) References cited:
- WO-A1-2010/084481
- WO-A1-2016/033322
- WO-A2-2009/034452
- US-A1- 2014 142 025

## Description

### Introduction

This invention relates to a collagen scaffold for the delivery of bioactive agents.

### Background of the Invention

Current 'gold standard' treatment approaches to orthopaedic or soft tissue microbial infections use systemic delivery of intravenous (IV) and/or oral antibiotics (>6 weeks). However, often, sufficient levels of therapeutic antibiotics at the site of infection are not achieved due to poor tissue diffusion and an avascularised pathology. Poor vascularisation at the site of infection is typically the root of the problem as antibiotics penetrate poorly into devascularised or poorly vascularised soft tissue and/or bone, resulting in insufficient doses of antibiotics reaching the site of infection while long term parenteral administration of antibiotics that result in effective doses at the bone can cause clinical problems associated with systemic toxicity.

The presence of antibiotic resistant staphylococcal strains and the ever-increasing incidence of methicillin-resistant S. *aureus* (MRSA) infections further complicate the treatment of these infections. The pathogen can also evade the host immune system by intracellular invasion, as S. *aureus* infects osteoblast bone, epithelial and fibroblast skin cells. This causes cell death while dead/dying bone and skin cells have been shown to release viable S. *aureus* capable of infecting adjacent tissue.

This capability to hide and re-emerge is suggested as how S. *aureus* evades antibiotic treatment and becomes a persistent infection. It is for this reason that bacterial infection in orthopaedic surgery is associated with significant morbidity and poorly functioning outcomes. The treatment and prophylaxis of both bone and soft tissue infections thus remains a major challenge.

In severe chronic infection, surgical intervention to remove necrotic tissue is required, followed by direct antibiotic administration. For example, the treatment of osteomyelitis (OM) often incorporates both a medical and surgical approach and antibiotic treatment can surpass three months depending on infection severity and treatment stage. Accordingly, a combination of surgical debridement and antibiotic therapy can assist in the treatment of infection. However, the resulting significant bone loss/destruction can delay the bone regeneration process. In addition, the prolonged use of antibiotics for periods in excess of three months can compromise tissue regeneration. Similarly, in relation to wound infections and surgical site infections, soft tissue will not regenerate in the presence of an infection. Thus, treatment complications associated with OM are comparable to complications associated with soft tissue infection.

Accordingly, in cases of chronic infection where surgical debridement is necessary, a loss of host tissue can result. As a result, a crucial part of successful surgical treatment is adequate dead space management following the debridement of the infected tissue. Dead bone and soft tissue must be replaced by viable vascularised tissue and care must be taken to prevent recurrence of infection or tissue regeneration can fail to result. Known dead space management techniques vary according to the type of infection and the extent of dead space post debridement. For example, smaller defects are usually filled with antibiotic depots and covered with soft tissue while larger defects require aggressive treatment with a combination of free flaps, bone grafts or external fixation. Clinicians also administer antibiotics directly into the excised site as compensation for direct administration however this technique is not ideal as the antibiotic can leach away from the infected site and, furthermore, therapeutic doses of the antibiotic are not released over the required treatment period which can be up to eight weeks. A need therefore exists for devices and methods which facilitate the administration of optimal doses of antibiotics at dead spaces to control and eliminate infection whilst still allowing for tissue regeneration.

Accordingly, various specific techniques have been developed to manage dead spaces. For example, in a widely employed method, dead spaces are filled with beads fabricated from the bone cement, polymethylmethacrylate (PMMA). Septopal (Trade Mark) PMMA beads can be impregnated with various types and doses of antibiotics which elute into the dead space (post-surgical debridement of infected tissue) to assist in eradicating any remaining infection or to act as a prophylaxis against re-colonisation. However, while some of the antibiotics are eluted, the majority remains trapped within the non-biodegradable beads. In addition, a second procedure is required to remove the beads which necessitates removing healthy viable tissue that has grown around the PMMA beads causing the patient further discomfort and a recurrence of infection.

In order to avoid a second procedure to remove PMMA beads, biodegradable options have been developed to manage the surgical dead space for both bone and wound infections. Two popular treatments approved for human use are calcium sulphate (CaSO₄) beads or collagen sponges both of which can be impregnated with various antibiotics used in the treatment of microbial infections, such as moxifloxacin, daptomycin, fusidic acid, tobramycin, and vancomycin at different dosages allowing for local elution. Such products generally try to control antimicrobial release by freeze drying the antibiotic into collagen or by incorporating less soluble forms of antibiotic into the products. Nevertheless, the known products still fail to provide the antibiotic release kinetics required to fight infection for the prescribed treatment regime for antibiotics of eight weeks.

For example, where antibiotic laden CaSO₄ pellets have been used as a biodegradable bone graft substitute, antibiotic delivery has been shown to be poor, with the majority of antibiotics rapidly eluted within 24 hours which, as indicated above, does not provide adequate coverage for the long post-operative antibiotic regime required for chronic OM and the like. CaSO₄ pellets are also unsuitable as load-bearing spacers as they quickly lose mechanical function due to the rapid rate of resorption.

Collagen sponges are the most widely used and established biodegradable fillers as they have superior biocompatibility, are resorbed completely, non-toxic, and cheap. They are typically combined with gentamicin, a broad spectrum antibiotic, and are also able to deliver a greater amount of antibiotics compared to PMMA beads. However, similar to CaSO₄ pellets, they have been shown to release 94% of their antibiotic load within the first 24 hours and prolonged release is not guaranteed. The antibiotic collagen sponges also demonstrate poor load bearing capabilities.

US Patent Specification No. 2014/0142025 describes a crosslinked collagen construct which provides the immediate and sustained release of an antimicrobial in the form of silver which is present in the construct in an unbound form to provide immediate release and is also chemically bound to the construct for sustained release. The chemically bound silver is crosslinked to the collagen using nordihydroguaiaretic acid so that sustained release of the silver is achieved in vivo. However, the sustained release of the antimicrobial silver is not controlled and occurs regardless of microbial activity i.e. even where no infection is present which can impede tissue regeneration. Similarly, the dosage and immediate release of the unbound silver cannot be easily controlled as the unbound silver is distributed throughout the collagen construct. PCT Patent Specification No. 2016/033322 describes a multi-layered collagen scaffold. However, the document fails to disclose a first layer comprising an antimicrobial for immediate release and a second layer comprising an anti-microbial for sustained release and suffers from the same disadvantages as US Patent Specification No. 2014/0142025. Similarly, PCT Patent Specification No. 2010/084481 also describes a multi-layer collagen scaffold. However, as before, PCT Patent Specification No. 2010/084481 does not contemplate a first layer comprising an antimicrobial for immediate release and a second layer comprising an anti-microbial for sustained release and does not contemplate a collagen scaffold having immediate and sustained release antimicrobials in separate layers that exhibit a tailored release responsive to antimicrobial activity.

In summary, known antibiotic dead space fillers suffer from suboptimal antibiotic elution. Known biodegradable fillers elute the majority of their antibiotics within 24 hours of insertion or suffer from a lack of load bearing abilities and poor drug release profiles while in the case of PMMA beads, the majority of the antibiotic is trapped within the beads. Furthermore, while PMMA beads have weight bearing capabilities, they are non-biodegradable, thus requiring a second procedure to remove them. Where known collagen constructs and scaffolds are employed, antimicrobial elution is also suboptimal and uncontrolled. Moreover, even where multilayer collagen scaffolds are employed, immediate and sustained release antimicrobial layers are not employed with the result that antimicrobial elution cannot be easily tailored. Furthermore, sustained release of antimicrobials is not responsive to the presence of infection.

An object of the invention is to overcome at least some of the problems of the prior art.

### Summary of the Invention

The scope of this invention is defined by the claims. Embodiments in the description relating to methods of treatment are not covered by the claims.

According to the invention there is provided a collagen scaffold comprising:
a first collagen matrix layer and
a second collagen matrix layer wherein the first collagen matrix layer comprises a first bioactive agent physically entrapped in the first collagen matrix layer and releasable in a burst from the first collagen matrix layer, and
the second collagen matrix layer comprises a second bioactive agent chemically attached to the second collagen matrix layer and releasable in a sustained release from the second collagen matrix layer in response to the presence of microbes or a microbial enzyme.

According to the claimed invention, the second bioactive agent is releasable in response to the presence of microbes. Preferably, the second bioactive agent is releasable in response to a microbial enzyme. More preferably, the microbial enzyme is a protease. Most preferably, the protease is a collagenase.

Preferably, the second bioactive agent is crosslinked to the second collagen matrix layer. More preferably, the second bioactive agent is covalently crosslinked to the second collagen matrix layer.

Optionally, the first and second bioactive agents can be the same or different.

In a preferred embodiment of the invention, the first and/or second bioactive agents comprise an antimicrobial. Preferably, the antimicrobial comprises an antibiotic or an antifungal. More preferably, the antibiotic comprises free amine and/or carboxylic groups for chemical attachment to the second collagen matrix layer.

In one embodiment, the antibiotic is chemically attached to the second collagen matrix layer at an amide bond.

In a preferred embodiment of the invention, the antibiotic is selected from the group consisting of vancomycin, gentamycin and teicoplanin.

Advantageously, the first collagen matrix layer and/or the second collagen matrix layer comprises hydroxyapatite (HA) to form a collagen-hydroxyapatite (CHA) scaffold. Alternatively, the first collagen matrix layer and/or the second collagen matrix layer comprises glycosaminoglycans to form a collagen-glycosaminoglycans (CG) scaffold.

In any embodiment of the invention, the first collagen matrix layer is integrated with the second collagen matrix layer.

Suitably, the first and/or second collagen matrix layers comprise lyophilized collagen matrix layers.

Advantageously, the second collagen matrix layer is lyophilized to the first collagen matrix layer.

The invention also extends to a process for producing a multilayer collagen scaffold comprising:
forming a first collagen matrix layer having a chemically attached first bioactive agent to be releasable in a sustained release from the first collagen matrix layer in response to the presence of microbes or a microbial enzyme; and
forming a second collagen scaffold matrix layer having a physically entrapped second bioactive agent on the first collagen matrix layer to produce the multilayer collagen scaffold.

Preferably, the first collagen matrix layer is formed by physically entrapping the first bioactive agent in the first collagen matrix layer and chemically attaching the physically entrapped first bioactive agent to the first collagen matrix layer.

In one embodiment, the first bioactive agent is chemically attached to the first collagen matrix layer with a crosslinker. Preferably, the first bioactive agent is covalently attached to the first collagen matrix layer. More preferably, the first bioactive agent is covalently attached to the first collagen matrix layer at an amide bond. Most preferably, the crosslinker comprises 1-Ethyl-3-(3-Dimethlamniopropyl)-carbodiimide (EDAC).

Advantageously, the first and second collagen matrix layers are formed by lyophilization.

Preferably, the second collagen matrix layer is formed on the first collagen matrix layer by freeze drying the second collagen matrix layer on the first collagen matrix layer.

Advantageously, the first collagen matrix layer and/or the second collagen matrix layer comprises HA to form a CHA scaffold. Alternatively, the first collagen matrix layer and/or the second collagen matrix layer comprises glycosaminoglycans to form a CG scaffold.

Optionally, the first and second bioactive agents can be the same or different.

In a preferred embodiment of the invention, the first and/or second bioactive agents comprise an antimicrobial. Preferably, the antimicrobial comprises an antibiotic or an antifungal. More preferably, the antibiotic comprises free amine and/or carboxylic groups. Most preferably, the antibiotic is selected from the group consisting of vancomycin, gentamycin and teicoplanin.

Also described herein, but not being part of the claimed invention, is a method of treating a subject comprising:
implanting a collagen scaffold in the subject wherein the collagen scaffold comprises a first collagen matrix layer and a second collagen matrix layer wherein the first collagen matrix layer comprises a first bioactive agent physically entrapped in the first collagen matrix layer and the second collagen matrix layer comprises a second bioactive agent chemically attached to the second collagen matrix layer
releasing the first active agent in a burst from the first collagen matrix layer and
releasing the second active agent in a sustained release from the second collagen matrix layer.

The second bioactive agent is released in response to the presence of microbes or in response to a microbial enzyme.

Suitably, the microbial enzyme is a protease. Preferably, the protease is a collagenase.

The first and second bioactive agents can be the same or different.

The first and/or second bioactive agents may comprise an antimicrobial. Preferably, the antimicrobial comprises an antibiotic or an antifungal.

Advantageously, the antibiotic comprises free amine and/or carboxylic groups for chemical attachment to the second collagen matrix layer.

Preferably, the antibiotic is chemically attached to the second collagen matrix layer at an amide bond.

In one embodiment, the antibiotic is selected from the group consisting of vancomycin, gentamycin and teicoplanin.

The multi-layer collagen scaffolds of the invention are "off-the-shelf" and ready to use products, that can have as a primary function the treatment of infection (e.g. microbial soft or bone tissue infections such as OM), that offer controlled release of bioactive agents such as antimicrobials (antibiotics, antifungals and the like) to the target site whilst also facilitating tissue regeneration i.e. by providing a multi-layer collagen scaffold with a first layer containing a bioactive agent for immediate release and a second layer containing a bioactive agent for sustained release, elution of the bioactive agents can be controlled and tailored. More particularly, as discussed further below, where the bioactive agent is an antimicrobial such as an antibiotic, the immediate dosage and release of the antibiotic in the first layer can be easily controlled for an optimal "burst" dose to optimally attack infections while the sustained dosage and release of antibiotic is responsive to the presence of microbes i.e. is responsive to microbial activity. As a result, sustained release of antimicrobial occurs should the initial burst release fail to completely eliminate infection.

For example, when incorporating an antibiotic for the treatment of infections such as OM, the kinetics of release of the antibiotic from the multi-layer collagen scaffold consists of an initial high concentration delivery of the antibiotic from the first collagen matrix layer to result in bacterial clearance in the area (a burst), followed by a sustained release for up to 8 weeks from the second collagen matrix layer in response to microbial activity, to prevent re-infection. Where infection is present, tissue regeneration is not compromised by the multi-layer collagen scaffolds of the invention.

As the biocompatible and biodegradable collagen scaffold backbone is composed of a porous collagen matrix, the collagen matrix can be modified as required to incorporate osteoinductive HA (i.e. to form a CHA scaffold) for enhanced bone tissue regeneration in addition to the primary antimicrobial functionality of the multi-layer scaffold or utilized either HA free or incorporated with glycosaminoglycans to form a CG scaffold for enhanced soft tissue regeneration also in addition to the to the primary antimicrobial functionality of the multi-layer scaffold.

The collagen scaffolds of the invention can also aid in dead space management within the defect after surgical debridement of necrotic tissue. Whilst the primary function of the multi-layered collagen scaffolds of the invention is the controlled elimination of infection, the combination of controlled antibiotic release and a collagen scaffold backbone, which has an ability to promote soft and bone tissue regeneration *in vivo* in different animal models, results in significantly improved, tailored and targeted infection eradication without compromising tissue regeneration compared to the currently available treatment options.

Moreover, the collagen scaffolds of the invention exhibit adaptive or responsive release kinetics whereby the degree of infection can inherently control the level of antibiotic release. For example, in a highly infected site, the microbes begin to release and induce host release of proteases such as collagenases which can liberate the scaffold-bound antibiotic by collagen degradation, thus leading to eradication of the infection and the facilitation of tissue regeneration
Therefore, unlike known biodegradable antibiotic delivery systems directed at antimicrobial treatment only, the multi-layer composite collagen scaffolds of the invention can serve a dual purpose: 1) to deliver antibiotics locally and responsively in accordance with the degree of infection in an immediate and sustained release manner 2) to enhance tissue regeneration within the infected area i.e. the invention combines the ability of bone and soft tissue repair scaffolds with the controlled local release of antimicrobials. Either when directly incorporated into scaffolds, or subsequently crosslinked to the scaffold, the release rate of antimicrobials from the scaffold is controlled in such a way as to enable an initial burst release to eradicate deep seated infections and a longer controlled release to prevent infection reoccurrence.

The multi-layer scaffolds of the invention can deliver requisite continued doses of antibiotic over time to the site of infection, eradicating the infection with prolonged release while aiding in tissue regeneration. The scaffolds of the invention provide an optimal treatment which can result in: (a) reduction in the overuse of antibiotics and thus microbial antibiotic resistance, (b) reduction of reoccurring infection, (c) the delivery of high local doses and responsive release of antibiotics without associated systemic toxicity, (d) improved tissue regeneration and (e) shortened hospital in-patient treatment times.

The invention therefore overcomes the problems associated with traditional infection treatment and, in some embodiments, can also promote effective tissue regeneration in a challenging environment.

The scaffolds of the invention also permit surgeons the ability to debride further into necrotic tissue in chronic infections with confidence in post-surgical tissue regeneration.

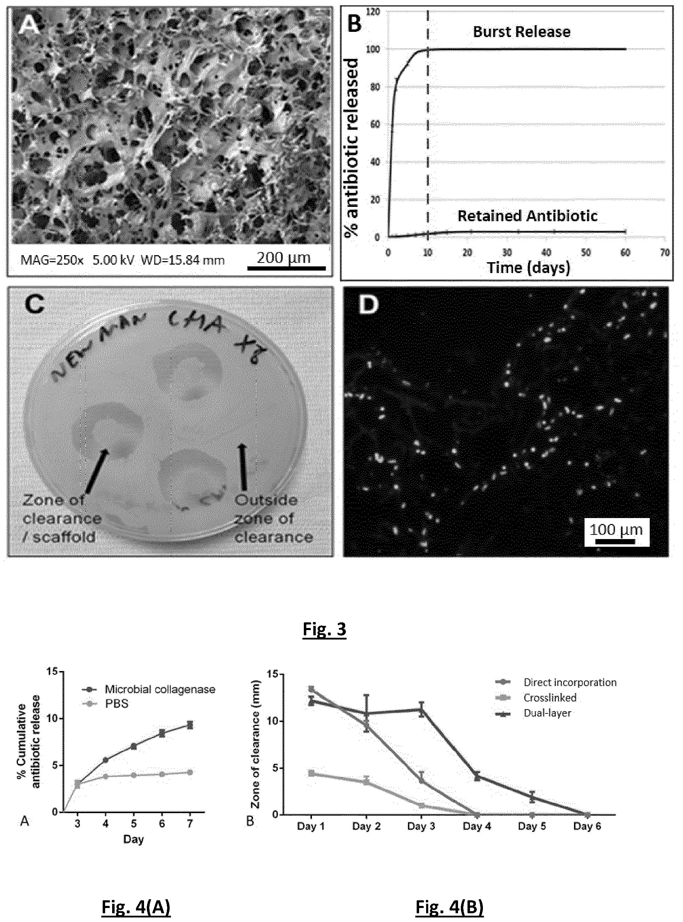

In the multi-layer scaffolds of the invention, a crosslinking agent such as 1-ethyl-3-(3-dimethylaminopropyl)carbodiimide hydrochloride) can be used to form an amide bond between the carboxyl and amine groups in the collagen and antibiotic components of collagen scaffolds, generally lyophilised multi-layer collagens scaffolds to achieve a sustained release of the antibiotic component from a crosslinked layer. The immediate or burst release in the collagen scaffold is achieved by using the layering process to add an additional non-crosslinked collagen/antibiotic layer to the crosslinked layer. In contradistinction, US Patent Specification No. 2014/142025 outlined above, describes the fabrication of a tubular collagen construct which contains an antimicrobial in the form of silver and is crosslinked using nordihydroguaiaretic acid (NDGA) which binds the silver to the construct allowing for sustained release. To achieve immediate release, additional silver is incorporated into the construct without chemical crosslinking. However, the immediate release silver and the sustained release silver are not in separate layers with the result that antimicrobial release cannot be tailored as with the present invention i.e. the separate layers of the present invention ensure that the make-up of the first layer can be formulated as required independently of the second layer e.g. in terms of antimicrobial type and dosage so that the immediate dosage and release of the antibiotic from the first layer can be selected for an optimal burst dose to optimally attack infections. Accordingly, improved burst release can be achieved by incorporating physically entrapped bioactive agents such as antibiotics into a separate layer as the release profile and mechanism can be designed independently of and separate to characteristics of the sustained release layer. In addition, the second sustained release layer of the multi-layer is responsive to microbial activity i.e. the covalent bonds formed when crosslinking the collagen and antibiotic components necessitate cleaving through the addition of microbial collagenase to allow the antibiotic to be released from the scaffold (see Fig. 4(A) described further below). Therefore, unlike the prior art, in the treatment of infected tissue, following the immediate release of antibiotic from the non-crosslinked layer, the antibiotic within the crosslinked layer is retained within the scaffold only to be released if; 1) the immediate antibiotic release was insufficient to kill the bacteria present or 2) there is a reoccurrence of infection. Accordingly, being separate, the burst release layer and the sustained release layers of the scaffolds of the invention can be independently optimized for optimal infection control.

Table 1 below summarises features and associated benefits of the collagen scaffolds of the invention.

**Table 1**

| **Feature** | **Benefit** |
|---|---|
| Multi-layer structure | • Bioactive agent profile of burst release layer can be easily controlled e.g. antibiotic and/or dosage for targeted immediate infection attack |
| | • Tailored sustained release of bioactive agent from sustained release layer responsive to microbial activity |
| Controlled local antibiotic delivery | • Dual local delivery and bactericidal |
| | • 1) Burst release (100% release at 10 days) |
| | • 2) Sustained release (<10% release at 60 days) |
| | • Reduces risk of systemic toxicity and non-cytotoxic microenvironment, does not affect host cell viability |
| Controlled delivery of different antibiotics | • Offers effectiveness against both gram-positive and gram-negative microbes |
| Biodegradable | • Resorbed into the body over time |
| Scaffolds for different applications | • Functionalised CG scaffold for wound / soft tissue infections |
| | • Functionalised CHA scaffold for bone infections |
| Enhanced Tissue Regeneration | • Proven capacity for bone and dermal regeneration |
| | • High porosity (> 98%) can facilitate optimal cell attachment and migration throughout scaffold |
| | • Increased cellular nutrient and waste exchange |

The dual-layer collagen-based scaffolds of the invention therefore make it possible to control the release of a tailored dose of active antimicrobials such as antibiotics from the collagen-based scaffolds. Two complementary approaches have been employed to achieve burst and sustained antimicrobial/antibiotic release - direct incorporation of the antimicrobials during the scaffold fabrication process (e.g. lyophilization), and chemical/cross-linking attachment of the antimicrobials to the scaffold post-fabrication.

The amount of antimicrobial such as antibiotic loaded into the scaffolds can be pre-determined and once released has an effective antibacterial activity without compromising cell viability so that tissue regeneration can take place. Therapeutic dosages of the antibiotics to be utilized in the dual-layer scaffolds of the invention can be pre-determined in a targeted manner for tissue with a high bacterial infection (e.g. 10⁸ bacteria cells/ml).

The scaffolds of the invention can also be fabricated without the use of, synthetic or semisynthetic polymers, chitosan and microspheres.

### Brief Description of the Drawings

The invention will now be described, by way of example only, with reference to the accompanying drawings and Examples in which:
Figure 1 is a schematic representation of a multi-layer scaffold in the form of a dual-layer collagen scaffold for the delivery of antibiotics made up of a sustained release layer (formed by crosslinking the antibiotic to the collagen scaffold resulting in chemical attachment) and a burst release layer (formed by the direct incorporation of antibiotic within the collagen scaffold resulting in physically entrapment) with a scanning electron microscopy (SEM) image of the scaffold showing the integrated sustained release and burst release layers;
Figure 2 is a schematic representation of the dual-layer collagen scaffold and an antibiotic release profile for the scaffold illustrating burst release for the directly incorporated antibiotic and sustained release for the crosslinked antibiotic;
Figure 3(A) is an SEM image of a dual-layer antibiotic (teicoplanin) eluting CHA scaffold;
Figure 3(B) are antibiotic release profiles from dual-layer scaffolds showing burst release via direct antibiotic incorporation and sustained release via crosslinking;
Figure 3(C) is an image of an agar plate seeded with *Staphylococcus aureus* showing the zone of clearance achieved by CHA scaffolds with antibiotics directly incorporated;
Figure 3(D) is a fluorescence micrograph of DAPI-stained osteoblasts seeded on antibiotic-eluting CHA scaffolds and infected with S. *aureus* in which the osteoblasts attach, remain viable, and migrate towards the scaffold center;
Figure 4(A) is a release profile of a crosslinked antibiotic-eluting scaffold with and without supplementation with microbial collagenase demonstrating a responsive increase in antibiotic release in the presence of microbial collagenase;
Figure 4(B) is a graphic illustration of agar diffusion tests performed with the dual layer scaffold of the invention against S. *aureus* compared with directly incorporated and crosslinked single layer scaffolds in which the dual-layer scaffold demonstrates enhanced bacterial clearance;
Figure 5(A) illustrates the results of a pre-clinical trial in a murine model of osteomyelitis in which treatment with the dual-layer scaffold resulted in lower levels of bacteria through the treatment period. Results are presented as mean ± standard error from mean. Significance; # p<0.1, * p<0.05;
Figure 5(B) illustrates the results of the pre-clinical trial in which bacteriological analysis of the animals following euthanasia demonstrated fewer infected animals in the dual-layer scaffold group. Data points represent CFU counts from individual animals. Line represents mean value of each group. Significance; # p<0.1 ;
Figure 5(C) is a representative micro-CT image analysis of a treated bone demonstrating evidence of mineralization within the defects treated with the dual-layer scaffold of the invention. Significance; # p<0.1, * p<0.05;
Figure 6(A) is graph of the bacteriological analysis after 4 weeks inoculation period in a pre-clinical trial in a rabbit model of chronic osteomyelitis. Data points represent CFU counts from individual animals. Line represents mean value of each group;
Figure 6(B) is a graph of the bacteriological analysis of the animals after the 8 week treatment period showing that infection was completely eradicated in the Gent dual-layer scaffold group. Data points represent CFU counts from individual animals. Line represents mean value of each group. Significance; * p<0.05;
Figure 7 is a graph of high performance liquid chromatography results demonstrating the percentage of Teicoplanin antibiotic released with increasing concentrations of crosslinker with optimal crosslinking being observed through drug release where the retention of the antibiotic cannot be further increased;
Figure 8(A) is a graph of the DNA content of control (antibiotic-free) dual-layer, vancomycin dual-layer and gentamicin dual-layer scaffolds seeded with MC3T3 cells from day 0 to day 21;
Figure 8(B) is a graph of the alkaline phosphatase (ALP) activity at day 7 and day 21 of cell-seeded scaffolds demonstrating similar levels of osteogenesis in each scaffold group;
Figure 8(C) are images of hematoxylin and eosin stained scaffolds at day 21 with the dashed line indicating the interface between the crosslinked and non-crosslinked layers of the scaffolds. Scale bar is 500 µm Significance; p< 0.05, a vs Day 0, b vs. Day 2, c vs. Day 7, d vs. Vane dual-layer, e vs. Gent dual-layer;
Figure 9(A) is a graph of the bone volume per total volume (BV/TV) of radial defects in a rabbit model of chronic osteomyelitis either left empty or treated with a Vane dual-layer scaffold or a Gent dual-layer scaffold;
Figure 9(B) is a graph of the bone density of defects at weeks 4, 8, 10 and 12, and
Figure 9(C) are CT reconstructions of radial defects at weeks 4, 8 10 and 12 showing bone healing in all groups over time. Scale bar is 3 mm. Significance: p<0.05; a vs Week 4, b vs. Week 8, c vs. Week 10, d vs. Vane dual-layer, e vs. Gent dual-layer.

### Detailed Description of the Invention

### Bioactive agents - Antimicrobials

In the following Examples, teicoplanin, vancomycin and gentamycin are shown to be suitable antibiotics for incorporation into the dual-layer collagen scaffolds as they are the clinical drugs of choice for the treatment of chronic infection caused by bacteria. However, the dual-layer collagen scaffolds of the invention allow the controlled delivery of other antibiotics with free amine and/or carboxylic groups that can be covalently cross-linked to the collagen. In addition, other effective and cost saving bioactive agents including antimicrobials such as antibiotics and antifungals for the treatment of fungal, gram positive and negative bacterial infections can also be employed in the dual-layer collagen scaffolds of the invention.

### Example 1 - Synthesis

Porous three-dimensional collagen-based scaffolds were fabricated following a well-established freeze-drying method (O'Brien et al., 2005). Briefly, for the typical fabrication of collagen scaffolds, the collagen was suspended in an acidic solution. If required, the collagen slurry solution can contain glycosaminoglycans for soft tissue regeneration (a CG scaffold), HA for bone regeneration (a CHA scaffold) or any other natural material that has suitable side chains for attachment of the antimicrobials.

The obtained slurry was then heavily degassed (to eliminate air bubbles that may generate non-controlled porosity) prior to adding the antimicrobial that possessed suitable attachment sites.

The antimicrobial was added to the collagen slurry using a syringe (direct incorporation) and injected through a needle (18G 1¹/₂") into the slurry, with gently mixing without introducing air bubbles. The collagen solution with the antimicrobial was then lyophilised by a freeze-drying process. In this process, the slurry was first frozen where both the final freezing temperature and the rate of freezing (ramp) were controlled to tune the resulting pore size. Next, the pressure was lowered to vacuum allowing the sublimation of ice crystals from the slurry to vapour resulting in the creation of a highly porous antibiotic-eluting collagen scaffold with an interconnected pore structure and homogenous pore size (O'Brien et al., 2005).

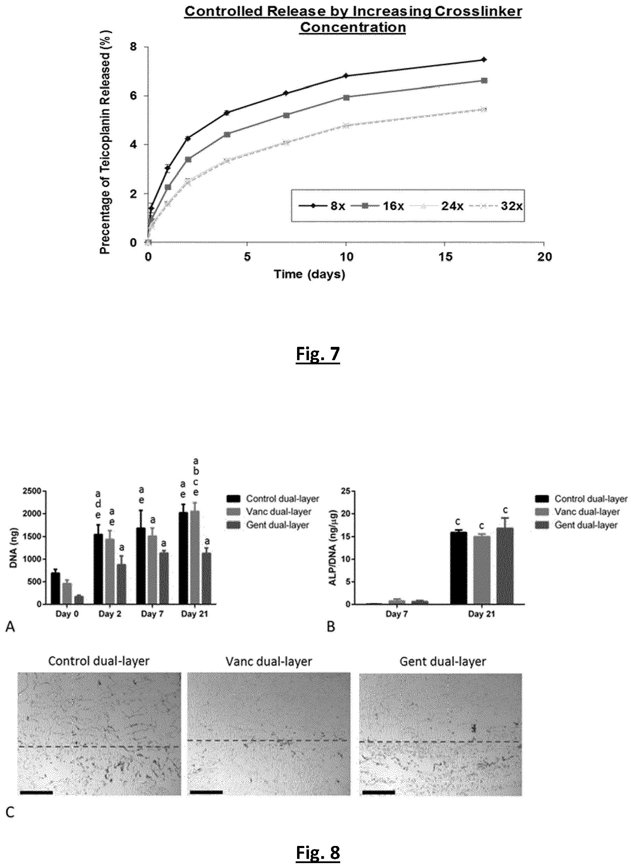

Freeze dried antibiotic-eluting collagen scaffolds were then crosslinked using 1-Ethyl-3-(3-Dimethlamniopropyl)-carbodimide hydrochloride (EDAC) and N-Hydroxysuccinimide (NHS) as a catalyst, resulting in the chemical attachment of the antibiotic to the collagen via covalent bonds. Briefly, collagen antibiotic scaffolds were immersed in a solution of EDAC/NHS, at increasing concentrations, to crosslink the antibiotic to the collagen. The EDAC was utilized at ranges from 8X to 32X the standard operating concentration (48 mM - 192 mM per gram of collagen). The crosslinked antibiotic-collagen scaffolds were then washed from all EDAC residues. Increasing the concentrations of the EDAC crosslinker resulted in the occupation of all the binding sites available between the antibiotic and the collagen based scaffold. As shown in Figure 7, this ultimately can slow the release of the antibiotic, achieving a controlled release system.

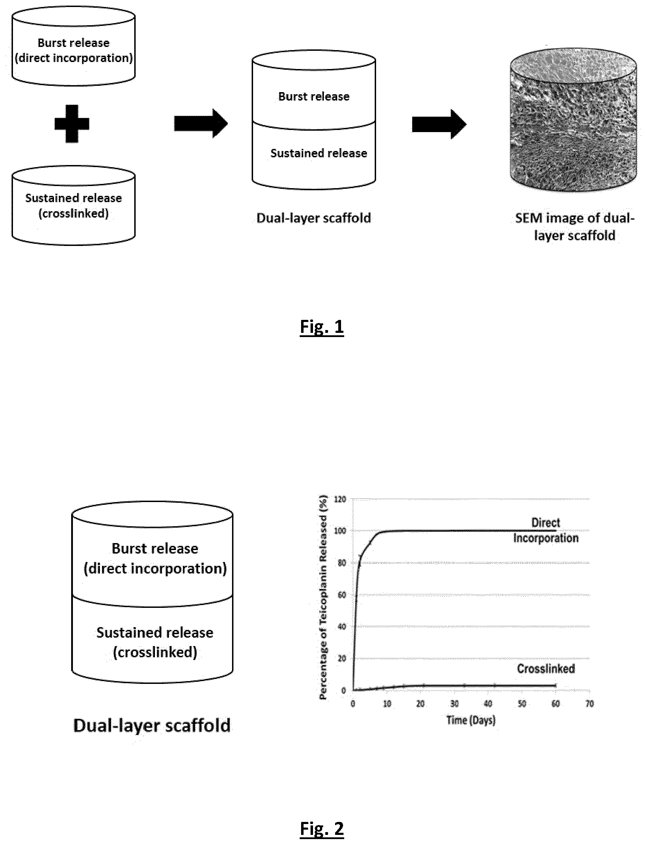

The dual-layered antibiotic-eluting collagen scaffold shown in Figure 1 was formed by placing a crosslinked antibiotic -eluting collagen scaffold (described above) on a stainless steel plate and pouring additional collagen slurry containing an antibiotic (directly incorporated) onto the top. The freeze drying process was then repeated to provide an inter connecting dual layered scaffold that contains antimicrobials to achieve a final antibiotic-eluting scaffold with a dual release profile.

The dual layered collagen scaffold can contain the same antimicrobial in each layer or two different antimicrobials as required.

The efficacy of the antibiotic-eluting dual-layer collagen scaffold was demonstrated in two pre-clinical trials.

### Example 2 - Pre-clinical Trial 1

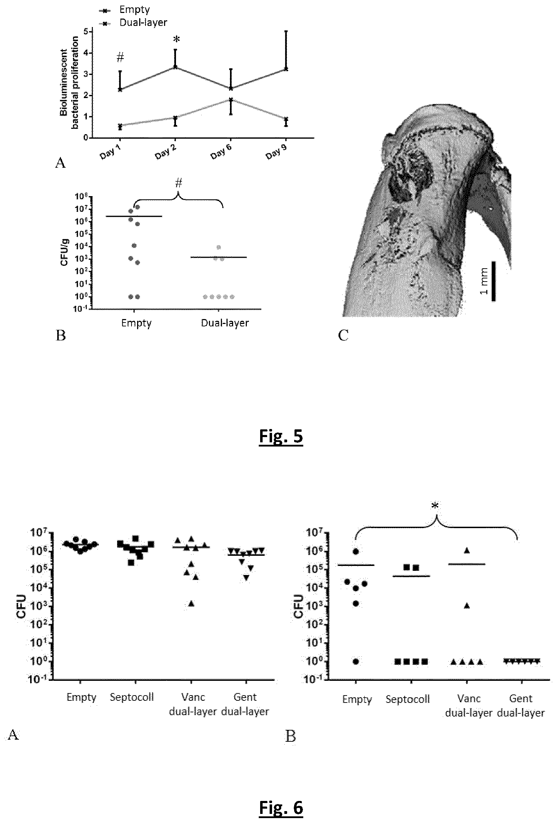

In pre-clinical trial 1, uni-cortical bone defects (Ø 0.8mm) in the tibiae of C57BL6 mice were inoculated with 2×10³ CFUs of bioluminescent *S.aureus.* After 2 weeks, the infected site was debrided (Ø 1.2mm) and either left empty or treated with a vancomycin-eluting dual-layer scaffold. Animals were sacrificed at day 11 posttreatment and tibiae were homogenized and counted for bacteria. Statistical comparisons were performed using t-tests. *In vivo* imaging during the treatment demonstrated lower levels of bioluminescent bacteria in scaffold treated animals compared to animals not treated with the scaffold (see Fig. 5(A)). Furthermore, bacteriological analyses of the animals at euthanasia demonstrated fewer infected animals in the scaffold treated group (see Fig. 5(B)). As shown in Fig. 5(C), micro-computed tomography (µCT) analysis also showed that the treated defects were beginning to fill with newly formed mineral.

### Example 3 - Pre-clinical Trial 2

Pre-clinical trial 2 utilised an animal model representative of chronic osteomyelitis. Infections were established in the radii of New Zealand White rabbits using inoculations of 2×10⁶ CFUs S. *aureus* over a period of 4 weeks. Following surgical debridement (6mm), rabbits then underwent treatment for a period of 8 weeks until euthanasia. The treatment groups were: 1) empty, 2) commercially available gentamicin-eluting collagen fleece (Septocoll E (Trade Mark)), 3) vancomycin-eluting dual-layer collagen scaffold (Vane dual-layer) and 4) gentamicin-eluting dual-layer collagen scaffold (Gent dual-layer). During the treatment period, all groups received systemic antibiotics (Cefazolin 25mg/kg), administered subcutaneously, twice daily, for 4 weeks. Results showed that inoculation resulted in the development of a sequestrum containing S. *aureus* in all rabbit radii, demonstrating the successful establishment of OM (see Fig. 6(A)). After the 8 week treatment period, only 1/6 (one out of six) rabbits in the empty group were infection-free, indicating that systemic antibiotic administration following debridement was insufficient to treat the infection. Infections were cleared in 4/6 rabbits in both the Septocoll E (Trade Mark) group and the Vane dual-layer collagen group. However, the most effective treatment was found to be the Gent dual-layer scaffold, which cleared the infection in 6/6 rabbits, a statistically significant improvement (p=0.0152) over the empty group (see Fig. 6(B)).

### Characterisation

As shown in Figure 3(A), in the dual-layer scaffold structure incorporating crosslinked and directly incorporated antibiotic produced as described above, the porous scaffold structure necessary for efficient tissue regeneration was maintained. In addition, the porosity (>98%) and mean pore size, essential for optimal proliferation and differentiation of osteoblasts during bone regeneration, were not compromised after the loading of the drug into the collagen structure. Depending on the incorporation method, different release kinetics of the antibiotic from the materials were obtained. Figure 3(B) shows the different delivery profiles obtained from different antibiotic-loaded materials for 60 days. At day 10, approximately 90% of the teicoplanin was released when it was directly loaded into the slurry during the CHA scaffold fabrication.

The chemical attachment of antibiotic onto the scaffold, achieved by the chemical crosslinkers that generated a stable amide bond between the drug and the collagen component of the scaffold, resulted in the retention of the drug within the scaffold. This ultimately resulted in a sustained release of the antibiotic, with approximately 11% teicoplanin released at day 60.

Furthermore, as shown in Figure 4(B) when crosslinked and direct incorporation scaffolds were layered to create a dual-layer scaffold, this dual-layer scaffold demonstrated synergistically enhanced bacterial clearance when compared to crosslinked or direct incorporation scaffolds alone.

Moreover, bacterial enzymes such as microbial collagenases were able to break the chemical bond between the collagen and the antibiotic, leading to the responsive increased release of the drug in the presence of S. *aureus* or S. *epidermidis.* This was demonstrated by the zone of clearance achieved after the culture of these bacteria (10⁸ colony forming units) in the presence of crosslinked scaffolds as shown in Figure 3(C).

The responsive release of antibiotic-loaded crosslinked scaffolds was further characterized by loading the scaffolds with a fluorescently tagged vancomycin antibiotic. As shown in Figure 4(A) when microbial collagenase was added to the antibiotic-eluting crosslinked scaffolds, an increase in antibiotic release rate was observed confirming that, once implanted into an infected environment, the scaffolds of the invention facilitate enhanced release of the antibiotic when it is most needed.

Accordingly, the dual-layer collagen scaffolds of the invention provide a microbially responsive release, whereby increased bacterial presence causes a responsive spike in the release of antibiotics from the scaffold leading to eradication of the infection.

Importantly, the antibiotic loaded scaffolds of the invention also demonstrate good cell compatibility. As shown in Figure 3(D), results achieved from the *in vitro* assessment of osteoblasts infected and cultured with live S. *aureus* on the antibiotic-eluting scaffolds of the invention demonstrated that osteoblast cells attached, remained viable, and migrated towards the centre of the antibiotic-eluting scaffolds. In contrast, parallel tests carried out with non-eluting materials identified that cells do not survive on scaffolds post-infection.

*In vitro* data sets demonstrated that 1) it is possible to load and release high doses of antibiotics from collagen based scaffolds in a controlled fashion while keeping the structural properties of these materials, 2) the released antibiotic retains antibacterial activity 3) the fabricated materials are cytocompatible and allow osteoblast and stem cell survival, attachment and migration and 4) the antibiotic-loaded crosslinked scaffolds are responsive to microbial activity.

As shown particularly in Figure 5(C), the scaffolds of the invention can also provide an antimicrobial platform in optimal tissue regeneration compared to commercially available collagen sponges/fleeces without compromising optimal scaffold physiochemical properties.

The release of antibiotics is controlled through either chemically crosslinking the antibiotic to the collagen backbone and/or allowing free diffusion by direct incorporation of the antibiotic i.e. a burst release. Different antimicrobial(s) can be chemically attached to the scaffold as required for a controlled sustained release with ~ 11% release over 2 months.

Moreover, as shown in the SEM image of Figure 1 (and in Figure 8(C)), the multi-layer scaffold of the invention does not contain barriers between the layers but is made up of seamlessly integrated layers so that 1) cell migration, 2) antibiotic diffusion, and 3) nutrient exchange / waste removal can continue unhindered. As a result, the multi-layer collagen scaffold of the invention is a unitary barrier-free scaffold containing a dual release system.

As shown in Figure 4(A), the scaffolds of the invention function as reservoirs of antibiotics with the ability to release more antibiotic in response to a relapsing infection. Each layer of the dual-layer scaffold can incorporate the same or different bioactive agents such as antimicrobials e.g. two or more different antibiotics can be included in a multilayer scaffold. Moreover it is possible to optimise dosage of therapeutic antimicrobials that does not harm host cells or tissue, while maintaining a high microbial activity. The scaffold structure can therefore be created as required as a multilayered scaffold which allows the ability to combine a burst and sustained release profile at the same time, with the ability to contain a choice of selected antimicrobial(s).

As indicated above, the scaffolds of the invention facilitate the control of the release kinetics of antimicrobials in such a way as to enable an initial burst release to eradicate deep seated infections, and a longer controlled release to prevent infection reoccurrence.

Figure 8(A) shows a graph of the DNA content of a control (antibiotic-free) dual-layer, vancomycin dual-layer and gentamicin dual-layer scaffolds seeded with MC3T3 cells. The cell seeded scaffolds were cultured in expansion medium for 2 days before being switched into an osteogenic medium for the duration of the experiment (21 days total). As shown in the graphs, all scaffolds demonstrated the capacity to promote proliferation of MC3T3 cells. Figure 8(B) shows a graph of the alkaline phosphatase (ALP) activity of the cell-seeded scaffolds demonstrating similar levels of osteogenesis in each scaffold group while Figure 8(C) shows images of hematoxylin and eosin staining of the scaffolds at day 21. As shown in the images, the dashed line indicates the interface between the crosslinked and non-crosslinked layers of the scaffolds. The excellent integration of the separate layers with no evidence of delamination is clearly visible in the image. Accordingly, despite being separate, the first and second collagen matrix layers form a seamless collagen scaffold that maintains its structural integrity in use.

As shown in Figure 6 described above, multi-layer scaffolds of the invention are extremely effective at eliminating infection which is the primary objective and function of the scaffolds. As indicated above, the vancomycin dual layer scaffold eliminated infection in 4/6 animals and the gentamicin dual layer scaffold eliminated infection in 6/6 animals. However, a secondary objective of the scaffolds of the invention can be to facilitate bone tissue healing unhindered and Figures 8(A) to 8(C) therefore demonstrate the capacity of the vancomycin and gentamicin scaffolds of the invention to facilitate mammalian cell attachment, proliferation, and osteogenic differentiation in vitro. Despite antibiotics being known to have the potential to be toxic to mammalian cells, the results demonstrate that the scaffolds of the present invention did not inhibit osteogenesis (as demonstrated by the ALP/DNA data in Figure 8(B)) in either vancomycin dual layer or gentamicin dual layer scaffolds compared to antibiotic-free dual layer scaffolds. Furthermore, although gentamicin dual layer scaffolds demonstrated a lower capacity to facilitate initial cell attachment, cell proliferation was subsequently observed to occur over time.

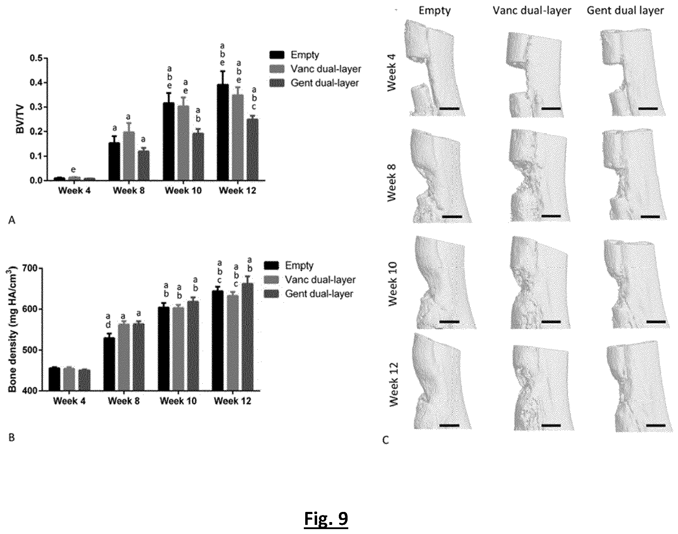

Figures 9(A) to 9(C) also demonstrate that bone tissue regeneration was not hindered by the collagen scaffolds of the invention (i.e. bone healing was visible in all groups over time) with Figure 9(A) being a graph of the bone volume per total volume (BV/TV) of radial defects in a rabbit model of chronic osteomyelitis either left empty or treated with a Vane dual-layer scaffold or a Gent dual-layer scaffold, Figure 9(B) being a graph of the bone density of defects at weeks 4, 8, 10 and 12, and Figure 9(C) showing CT reconstructions of radial defects at weeks 4, 8 10 and 12.

As shown in the drawings, the bone healing observed between 4 and 12 weeks in the empty group demonstrates that the size of the defect in this particular model is sub-critical and will heal itself over time. Although lower levels of BV/TV were observed in the gentamicin dual-layer group at 12 weeks compared to the empty group at 12 weeks, the results demonstrate that the primary objective of infection elimination was achieved while bone healing was still also achieved over time albeit at a lower rate i.e. an increase in BV/TV is still observed over time in the gentamicin dual-layer which demonstrates that bone healing is occurring, albeit at a slightly reduced rate.

In summary, in the collagen scaffolds of the invention, the physically entrapped antimicrobial in the first collagen matrix layer is released in an initial burst in vivo to attack and eliminate infection. Where the initial burst fails to eradicate the infection, the antimicrobials attached to the collagen matrix in the second layer via a crosslinking method to generate a drug-collagen covalent bond that can be broken by microbial infection are released via an adaptive release kinetic technology whereby the level of antibiotic release from the second layer is controlled by the degree of infection. Accordingly, in a highly infected site the bacteria begins to induce release of enzymes such as proteases which can liberate the collagen-bound antibiotic reservoir, thus leading to eradication of the infection and allowing the micro-environment necessary for tissue regeneration.

## Claims

1. A collagen scaffold comprising:
a first collagen matrix layer and
a second collagen matrix layer wherein the first collagen matrix layer comprises a first bioactive agent physically entrapped in the first collagen matrix layer and releasable in a burst from the first collagen matrix layer, and the second collagen matrix layer comprises a second bioactive agent chemically attached to the second collagen matrix layer and releasable in a sustained release from the second collagen matrix layer in response to the presence of microbes or a microbial enzyme.

2. A collagen scaffold as claimed in Claim 1 wherein the microbial enzyme is a protease.

3. A collagen scaffold as claimed in Claim 1 or Claim 2 wherein the second bioactive agent is crosslinked to the second collagen matrix layer.

4. A collagen scaffold as claimed in any of Claims 1 to 3 wherein the first and/or second bioactive agents comprise an antimicrobial.

5. A collagen scaffold as claimed in any of Claims 1 to 4 wherein the first collagen matrix layer and/or the second collagen matrix layer comprises hydroxyapatite to form a collagen-hydroxyapatite scaffold or glycosaminoglycans to form a collagen-glycosaminoglycans scaffold.

6. A collagen scaffold as claimed in any of Claims 1 to 5 wherein the first collagen matrix layer is integrated with the second collagen matrix layer.

7. A process for producing a multilayer collagen scaffold comprising:
forming a first collagen matrix layer having a chemically attached first bioactive agent to be releasable in a sustained release from the first collagen matrix layer in response to the presence of microbes or a microbial enzyme; and
forming a second collagen scaffold matrix layer having a physically entrapped second bioactive agent on the first collagen matrix layer to produce the multilayer collagen scaffold.

8. A process for producing a multilayer collagen scaffold as claimed in Claim 7 wherein first collagen matrix layer is formed by physically entrapping the first bioactive agent in the first collagen matrix layer and chemically attaching the physically entrapped first bioactive agent to the first collagen matrix layer.

9. A process for producing a multilayer collagen scaffold as claimed in Claim 8 wherein the first bioactive agent is chemically attached to the first collagen matrix layer with a crosslinker.

10. A process for producing a multilayer collagen scaffold as claimed in Claim 9 wherein the first bioactive agent is covalently attached to the first collagen matrix layer.

11. A process for producing a multilayer collagen scaffold as claimed in Claim 10 wherein the first bioactive agent is covalently attached to the first collagen matrix layer at an amide bond.

12. A process for producing a multilayer collagen scaffold as claimed in any of Claims 9 to 11 wherein the crosslinker comprises 1-Ethyl-3-(3-Dimethlamniopropyl)-carbodiimide (EDAC).

13. A process for producing a multilayer collagen scaffold as claimed in any of Claims 9 to 12 wherein the first and second collagen matrix layers are formed by lyophilization.

14. A process for producing a multilayer collagen scaffold as claimed in any of Claims 8 to 13 wherein the first collagen matrix layer and/or the second collagen matrix layer comprises hydroxyapatite to form a collagen-hydroxyapatite scaffold.

15. A process for producing a multilayer collagen scaffold as claimed in any of Claims 8 to 14 wherein:
the first collagen matrix layer and/or the second collagen matrix layer comprises glycosaminoglycans to form a collagen-glycosaminoglycans scaffold; and/or
the first and second bioactive agents can be the same or different; and/or
the first and/or second bioactive agents comprise an antimicrobial; and/or
the antimicrobial comprises an antibiotic or an antifungal; and/or
the antibiotic comprises free amine and/or carboxylic groups; and/or
the antibiotic is selected from the group consisting of vancomycin, gentamycin and teicoplanin.

## Patentansprüche

1. Kollagengerüst, das Folgendes umfasst:
eine erste Kollagenmatrixschicht und
eine zweite Kollagenmatrixschicht, wobei die erste Kollagenmatrixschicht einen ersten bioaktiven Wirkstoff umfasst, der physikalisch in der ersten Kollagenmatrixschicht eingeschlossen ist und in einem Ausbruch aus der ersten Kollagenmatrixschicht freisetzbar ist, und die zweite Kollagenmatrixschicht einen zweiten bioaktiven Wirkstoff umfasst, der chemisch an der zweiten Kollagenmatrixschicht angebracht ist und in einer anhaltenden Freisetzung aus der zweiten Kollagenmatrixschicht als Reaktion auf die Gegenwart von Mikroben oder eines mikrobiellen Enzyms freisetzbar ist.

2. Kollagengerüst nach Anspruch 1, wobei das mikrobielle Enzym eine Protease ist.

3. Kollagengerüst nach Anspruch 1 oder Anspruch 2, wobei der zweite bioaktive Wirkstoff mit der zweiten Kollagenmatrixschicht vernetzt ist.

4. Kollagengerüst nach einem der Ansprüche 1 bis 3, wobei der erste und/oder zweite bioaktive Wirkstoff ein antimikrobielles Mittel umfassen.

5. Kollagengerüst nach einem der Ansprüche 1 bis 4, wobei die erste Kollagenmatrixschicht und/oder die zweite Kollagenmatrixschicht Folgendes umfassen: Hydroxyapatit, um ein Kollagen-Hydroxyapatit-Gerüst zu bilden, oder Glycosaminoglycane, um ein Kollagen-Glycosaminoglycane-Gerüst zu bilden.

6. Kollagengerüst nach einem der Ansprüche 1 bis 5, wobei die erste Kollagenmatrixschicht mit der zweiten Kollagenmatrixschicht eingebunden ist.

7. Verfahren zur Herstellung eines mehrschichtigen Kollagengerüsts, das Folgendes umfasst:
Bilden einer ersten Kollagenmatrixschicht, die einen chemisch angebrachten, ersten bioaktiven Wirkstoff aufweist, um in einer anhaltenden Freisetzung aus der ersten Kollagenmatrixschicht als Reaktion auf die Gegenwart von Mikroben oder eines mikrobiellen Enzyms freisetzbar zu sein; und
Bilden einer zweiten Kollagengerüstmatrixschicht, die einen physikalisch eingeschlossenen zweiten bioaktiven Wirkstoff aufweist, auf der ersten Kollagenmatrixschicht, um das mehrschichtige Kollagengerüst herzustellen.

8. Verfahren zur Herstellung eines mehrschichtigen Kollagengerüsts nach Anspruch 7, wobei die erste Kollagenmatrixschicht durch physikalischen Einschluss des ersten bioaktiven Wirkstoffs in der ersten Kollagenmatrixschicht und chemische Anbringung des physikalisch eingeschlossenen ersten bioaktiven Wirkstoffs an der ersten Kollagenmatrixschicht gebildet wird.

9. Verfahren zur Herstellung eines mehrschichtigen Kollagengerüsts nach Anspruch 8, wobei der erste bioaktive Wirkstoff chemisch an der ersten Kollagenmatrixschicht mit einem Vernetzungsmittel angebracht wird.

10. Verfahren zur Herstellung eines mehrschichtigen Kollagengerüsts nach Anspruch 9, wobei der erste bioaktive Wirkstoff kovalent an der ersten Kollagenmatrixschicht angebracht wird.

11. Verfahren zur Herstellung eines mehrschichtigen Kollagengerüsts nach Anspruch 10, wobei der erste bioaktive Wirkstoff kovalent an einer Amidbindung an der ersten Kollagenmatrixschicht angebracht wird.

12. Verfahren zur Herstellung eines mehrschichtigen Kollagengerüsts nach einem der Ansprüche 9 bis 11, wobei das Vernetzungsmittel 1-Ethyl-3-(3-dimethylaminopropyl)carbodiimid (EDAC) umfasst.

13. Verfahren zur Herstellung eines mehrschichtigen Kollagengerüsts nach einem der Ansprüche 9 bis 12, wobei die erste und zweite Kollagenmatrixschicht durch Lyophilisierung gebildet werden.

14. Verfahren zur Herstellung eines mehrschichtigen Kollagengerüsts nach einem der Ansprüche 8 bis 13, wobei die erste Kollagenmatrixschicht und/oder die zweite Kollagenmatrixschicht Hydroxyapatit umfassen, um ein Kollagen-Hydroxyapatit-Gerüst zu bilden.

15. Verfahren zur Herstellung eines mehrschichtigen Kollagengerüsts nach einem der Ansprüche 8 bis 14, wobei:
die erste Kollagenmatrixschicht und/oder die zweite Kollagenmatrixschicht Glycosaminoglycane umfassen, um ein Kollagen-Glycosaminoglycane-Gerüst zu bilden, und/oder
der erste und zweite bioaktive Wirkstoff gleich oder verschieden sein können; und/oder der erste und/oder zweite bioaktive Wirkstoff ein antimikrobielles Mittel umfassen,
und/oder
das antimikrobielle Mittel ein Antibiotikum oder ein Antimykotikum umfasst, und/oder das Antibiotikum freie Amin- und/oder Carboxylgruppen umfasst, und/oder das Antibiotikum aus der Gruppe ausgewählt, die aus Vancomycin, Gentamycin und Teicoplanin besteht.

## Revendications

1. Echafaudage de collagène, comprenant
une première couche de matrice de collagène, et
une deuxième couche de matrice de collagène, où la première couche de matrice de collagène comprend un premier agent bioactif physiquement piégé dans la première couche de matrice de collagène et pouvant être libéré en décharge à partir de la première couche de matrice de collagène, et
la deuxième couche de matrice de collagène comprend un deuxième agent bioactif fixé chimiquement à la deuxième couche de matrice de collagène et pouvant être libéré par libération prolongée à partir de la deuxième couche de matrice de collagène en réponse à la présence de microbes ou d'une enzyme microbienne.

2. Echafaudage de collagène selon la revendication 1, dans lequel l'enzyme microbienne est une protéase.

3. Echafaudage de collagène selon la revendication 1 ou la revendication 2, dans lequel le deuxième agent bioactif est réticulé à la deuxième couche de matrice de collagène.

4. Echafaudage de collagène selon l'une quelconque des revendications 1 à 3, dans lequel le premier et/ou le deuxième agent bioactif comprend un agent antimicrobien.

5. Echafaudage de collagène selon l'une quelconque des revendications 1 à 4, dans lequel la première couche de matrice de collagène et/ou la deuxième couche de matrice de collagène comprend de l'hydroxyapatite afin de former un échafaudage de collagène-hydroxyapatite ou des glycosaminoglycanes afin de former un échafaudage de collagène-glycosaminoglycanes.

6. Echafaudage de collagène selon l'une quelconque des revendications 1 à 5, dans lequel la première couche de matrice de collagène est intégrée au sein de la deuxième couche de matrice de collagène.

7. Procédé de production d'un échafaudage de collagène multicouche, comprenant :
la formation d'une première couche de matrice de collagène ayant un premier agent bioactif chimiquement fixé, pour pouvoir être libéré selon une libération prolongée à partir de la première couche de matrice de collagène en réponse à la présence de microbes ou d'une enzyme microbienne ; et
la formation d'une deuxième couche de matrice d'échafaudage de collagène ayant un deuxième agent bioactif physiquement piégé sur la première couche de matrice de collagène afin de produire l'échafaudage de collagène multicouche.

8. Procédé de production d'un échafaudage de collagène multicouche selon la revendication 7, dans lequel la première couche de matrice de collagène est formée en piégeant physiquement le premier agent bioactif dans la première couche de matrice de collagène et en fixant chimiquement le premier agent bioactif physiquement piégé à la première couche de matrice de collagène.

9. Procédé de production d'un échafaudage de collagène multicouche selon la revendication 8, dans lequel le premier agent bioactif est fixé chimiquement à la première couche de matrice de collagène à l'aide d'un agent de réticulation.

10. Procédé de production d'un échafaudage de collagène multicouche selon la revendication 9, dans lequel le premier agent bioactif est fixé de manière covalente à la première couche de matrice de collagène.

11. Procédé de production d'un échafaudage de collagène multicouche selon la revendication 10, dans lequel le premier agent bioactif est fixé de manière covalente à la première couche de matrice de collagène au niveau d'une liaison amide.

12. Procédé de production d'un échafaudage de collagène multicouche selon l'une quelconque des revendications 9 à 11, dans lequel l'agent de réticulation comprend du 1-éthyl-3-(3-diméthylaminopropyl)carbodiimide (EDAC).

13. Procédé de production d'un échafaudage de collagène multicouche selon l'une quelconque des revendications 9 à 12, dans lequel la première et la deuxième couche de matrice de collagène sont formées par lyophilisation.

14. Procédé de production d'un échafaudage de collagène multicouche selon l'une quelconque des revendications 8 à 13, dans lequel la première couche de matrice de collagène et/ou la deuxième couche de matrice de collagène comprend de l'hydroxyapatite, afin de former un échafaudage de collagène-hydroxyapatite.

15. Procédé de production d'un échafaudage de collagène multicouche selon l'une quelconque des revendications 8 à 14, dans lequel :
la première couche de matrice de collagène et/ou la deuxième couche de matrice de collagène comprend des glycosaminoglycanes afin de former un échafaudage de collagène-glycosaminoglycanes ; et/ou
le premier et le deuxième agent bioactif peuvent être identiques ou différents ;
et/ou
le premier et/ou le deuxième agent bioactif comprennent un agent antimicrobien ; et/ou
l'agent antimicrobien comprend un antibiotique ou un antifongique ; et/ou
l'antibiotique comprend des groupements amine et/ou carboxylique libres ; et/ou
l'antibiotique est choisi dans le groupe constitué par la vancomycine, la gentamycine et la téicoplanine.
